# EUROPEAN PATENT APPLICATION

(11) **EP 2 893 808 A1**
(43) Date of publication of application: **15.07.2015**
(21) Application number: 13834929.5
(22) Date of filing: 03.09.2013
(51) Int. Cl.: A01N 43/653, A01N 25/00, A01P 21/00

(54) **PLANT GROWTH ACCELERATOR**

(30) Priority: 06.09.2012 JP 2012195757
(71) Applicant: Nippon Soda Co., Ltd., Tokyo 100-8165 (JP)
(72) Inventor: MAKITA, Satoru, Odawara-shi Kanagawa 250-0280 (JP)
(74) Representative: Carlisle, Julie
(86) International application number: PCT/JP2013/005204
(87) International publication number: WO 2014/038185

(57) **Abstract**

A plant growth accelerator or an agent for growing vigourous seedlings, which comprises a compound represented by formula (1) as an active ingredient: 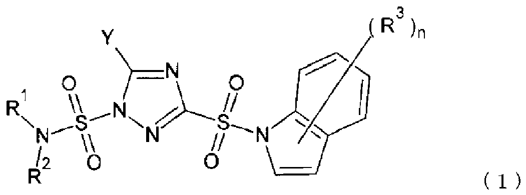 [wherein R¹ and R² each independently represent a C₁₋₄ alkyl group; Y represents a hydrogen atom, a halogeno group, a C₁₋₄ alkyl group, or a C₁₋₄ haloalkyl group; each R³ independently represents a halogeno group, a cyano group, a nitro group, a C₁₋₄ alkyl group or a C₁₋₄ haloalkyl group; and n represents the number of R³ and is an integer of 0 to 6.]

## Description

### Technical Field

The present invention relates to a plant growth accelerator or an agent for growing vigourous seedlings.

This application claims priority to Japanese Patent Application No. 2012-195757, filed on September 6, 2012, the disclosure of which is hereby incorporated by reference.

### Background Art

For example, the box seedling method of rice leads to a super-dense seeding due to being adapted to a rice transplanter. Thus, making a mistake in controlling temperature, humidity, and the like can result in the poor growth of rice seedlings and the reduced quality of seedlings. After being transplanted to a paddy field, the seedlings having reduced quality are easily affected by pests and pathogens and are less resistant to changes in weather conditions because of their poor survival properties. Thus, a yield decrease can occur in spite of having expended considerable cost and effort.

In response to such problems, for the purpose of improving the yield of plants, plant growth accelerators or agents for growing vigourous seedlings have been proposed which have effects, such as rooting acceleration, lodging prevention, cold tolerance improvement, lengthening leaf greenness period, vigourous seedling growth, an increase in the number of tillers, and organ growth acceleration.

Patent Document 1 describes a composition for promoting rooting and growing vigourous seedlings of rice, comprising a choline and hydroxyisoxazole. Patent Document 2 proposes an agent for growing vigourous seedlings of rice, comprising 3-hydroxypyrazine or its derivative as an active ingredient. Patent Document 3 describes that a particular tetrazoyloxime derivative is effective as an agent for growing vigourous seedlings of rice.

### Prior Art Documents

### Patent Documents

Patent Document 1: Japanese unexamined Patent Application Publication No. 61-212504
Patent Document 2: Japanese unexamined Patent Application Publication No. 62-167710
Patent Document 3: WO2010/001563
Patent Document 4: WO99/021851

### Summary of the Invention

### Object to be Solved by the Invention

An object of the present invention is to provide a plant growth accelerator or an agent for growing vigourous seedlings which is new and has an excellent efficacy.

### Means to Solve the Object

As a result of studies for solving the above-described problems, the present inventor has found that a certain sulfamoyl compound has a growth acceleration effect or the effect of growing vigourous seedlings. The present invention has been accomplished based on the finding.

Thus, the present invention encompasses the following aspects.
[1] A plant growth accelerator comprising a compound represented by formula (1) as an active ingredient. 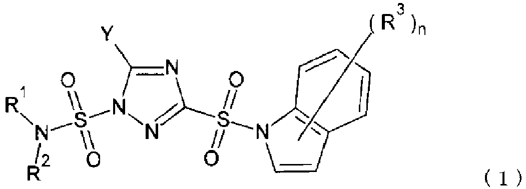 [wherein R¹ and R² each independently represent a C₁₋₄ alkyl group; Y represents a hydrogen atom, a halogeno group, a C₁₋₄ alkyl group, or a C₁₋₄ haloalkyl group; each R³ independently represents a halogeno group, a cyano group, a nitro group, a C₁₋₄ alkyl group, or a C₁₋₄ haloalkyl group; and n represents the number of R³ and is an integer of 0 to 6.]
[2] A plant growth accelerator comprising 3-(3-bromo-6-fluoro-2-methylindol-1-ylsulfonyl)-N,N-dimethyl-1H-1,2,4-triazole-1-sulfonamide as an active ingredient.
[3] The plant growth accelerator according to [1] or [2], wherein the accelerator is for seed treatment.
[4] An agent for growing vigourous seedlings, comprising a compound represented by formula (1) as an active ingredient. 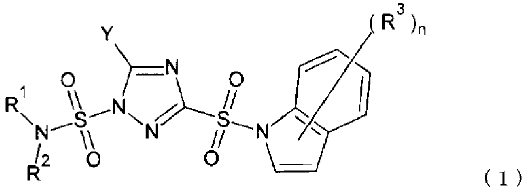 [wherein R¹ and R² each independently represent a C₁₋₄ alkyl group; Y represents a hydrogen atom, a halogeno group, a C₁₋₄ alkyl group, or a C₁₋₄ haloalkyl group; each R³ independently represents a halogeno group, a cyano group, a nitro group, a C₁₋₄ alkyl group, or a C₁₋₄ haloalkyl group; and n represents the number of R³ and is an integer of 0 to 6.]
[5] An agent for growing vigourous seedlings, comprising 3-(3-bromo-6-fluoro-2-methylindol-1-ylsulfonyl)-N,N-dimethyl-1H-1,2,4-triazole-1-sulfonamide as an active ingredient.
[6] The agent for growing vigourous seedlings according to [4] or [5], wherein the agent is for seed treatment.
[7] A method for using a compound represented by formula (1) for the purpose of plant growth acceleration. 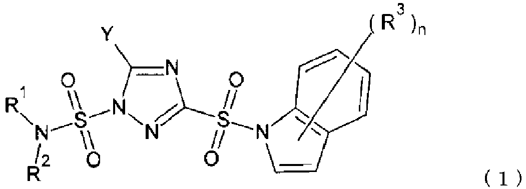 [wherein R¹ and R² each independently represent a C₁₋₄ alkyl group; Y represents a hydrogen atom, a halogeno group, a C₁₋₄ alkyl group, or a C₁₋₄ haloalkyl group; each R³ independently represents a halogeno group, a cyano group, a nitro group, a C₁₋₄ alkyl group, or a C₁₋₄ haloalkyl group; and n represents the number of R³ and is an integer of 0 to 6.]
[8] A method for using a compound represented by formula (1) for the purpose of growing vigourous seedlings. 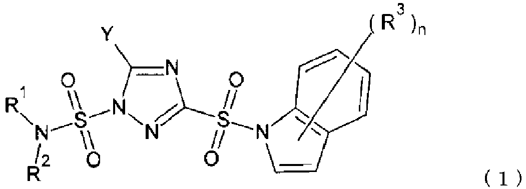 [wherein R¹ and R² each independently represent a C₁₋₄ alkyl group; Y represents a hydrogen atom, a halogeno group, a C₁₋₄ alkyl group, or a C₁₋₄ haloalkyl group; each R³ independently represents a halogeno group, a cyano group, a nitro group, a C₁₋₄ alkyl group, or a C₁₋₄ haloalkyl group; and n represents the number of R³ and is an integer of 0 to 6.]

### Effect of the Invention

The plant growth accelerator or the agent for growing vigourous seedlings according to the present invention has an excellent growth accelerating effect or effect of growing vigourous seedlings. The application of the plant growth accelerator or the agent for growing vigourous seedlings according to the present invention to seed or seedlings before transplantation can be expected to have the effects of increasing the taking root of seedlings after the transplantation, also increasing the number of tillers, and particularly enhancing resistance to low-temperature injury. The accelerator or the agent also accelerates the growth of the above-ground parts of seedlings, which can be expected to shorten the period necessary for growth and enhance the yield. In addition, the accelerator or the agent also exerts the effect of preventing the occurrence of damping-off.

### Mode of Carrying Out the Invention

For the purpose of the present invention, the plant growth acceleration effect refers to the effect of increasing the rate or speed of germination from seeds, accelerating rooting, elongating and thickening stems or roots, tillering, developing and elongating leaves, increasing the percentage of anthesis and seed set, thickening fruits, or accelerating the degree and/or speed of the growth of a part of or all of a plant body including these parts; the plant growth accelerator refers to an agent having the effect.

For the purpose of the present invention, the effect of growing vigourous seedlings refers to the effect of vigourously growing the seedlings of a plant; the agent for growing vigourous seedlings refers to an agent having the effect.

The effect of growing vigourous seedlings also encompasses the effects of accelerating rooting and the growth of seedling roots or the whole seedling; however, it also encompasses the effect of vigourous growing seedlings by preventing spindly growth (the only undue elongation of an above-ground stem without thickening), lodging, wilting, and the like, or enhancing cold tolerance, lengthening leaf greenness period, and the like.

The plant growth accelerator or the agent for growing vigourous seedlings according to the present invention comprises a compound represented by formula (1) as an active ingredient. The compound represented by formula (1) is a substance known as an agricultural or horticultural fungicide (Patent Document 4). The method for its production is described, for example, in Patent Document 4.

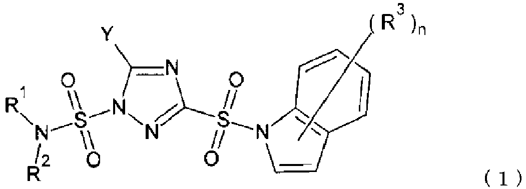

In formula (1), R¹ and R² each independently represent a C₁₋₄ alkyl group.

Examples of the C₁₋₄ alkyl group for R¹ and R² include a methyl group, an ethyl group, an n-propyl group, an i-propyl group, an n-butyl group, an s-butyl group, an i-butyl group, and a t-butyl group.

In formula (1), Y represents a hydrogen atom, a halogeno group, a C₁₋₄ alkyl group, or a C₁₋₄ haloalkyl group.

Examples of the halogeno group for Y include a fluoro group, a chloro group, a bromo group, and an iodo group.

Examples of the C₁₋₄ alkyl group for Y include the same as those for R¹ and R².

Examples of the C₁₋₄ haloalkyl group for Y include a chloromethyl group, a fluoromethyl group, a dichloromethyl group, a difluoromethyl group, a dichlorofluoromethyl group, a trifluoromethyl group, a pentafluoroethyl group, and a 3,3,3-trifluoro-n-propyl group.

In formula (1), each R³ independently represents a halogeno group, a cyano group, a nitro group, a C₁₋₄ alkyl group, or a C₁₋₄ haloalkyl group. n represents the number of R³ and is an integer of 0 to 6. When a plurality of R³ are present, they can be the same or different from each other.

Examples of the halogeno group for R³ include the same as those for Y.

Examples of the C₁₋₄ alkyl group for R³ include the same as those for R¹ and R².

Examples of the C₁₋₄ haloalkyl group for R³ include the same as those for Y.

In view of availability and the like, the compound represented by formula (1) is preferably 3-(3-bromo-6-fluoro-2-methylindol-1-ylsulfonyl)-N,N-dimethyl-1H-1,2,4-triazole-1-sulfonamide (general name: amisulbrom).

The plant growth accelerator or the agent for growing vigourous seedlings according to the present invention can comprise additives commonly used in preparations, in addition to a compound represented by formula (1). Additives include a solid carrier, a liquid carrier, a dispersant, a diluent, an emulsifier, a spreader, and a thickener.

Examples of the solid carrier or the liquid carrier include talc, clay, bentonite, kaolin, diatomaceous earth, montmorillonite, mica, vermiculite, gypsum, calcium carbonate, white carbon, wood flour, starch, alumina, a silicate, a glycopolymer, a wax, water, an alcohol (e.g., methyl alcohol, ethyl alcohol, n-propyl alcohol, isopropyl alcohol, n-butyl alcohol, ethylene glycol, or benzyl alcohol), a petroleum fraction (e.g., petroleum ether, kerosene, or solvent naphtha), an aliphatic or alicyclic hydrocarbon (e.g., n-hexane or cyclohexane), an aromatic hydrocarbon (e.g., benzene, toluene, xylene, ethylbenzene, chlorobenzene, cumene, or methylnaphthalene), a halogenated hydrocarbon (e.g., chloroform or dichloromethane), an ether (e.g., isopropyl ether, ethylene oxide, or tetrahydrofuran), a ketone (e.g., acetone, methyl ethyl ketone, cyclohexanone, or methyl isobutyl ketone), an ester (e.g., ethyl acetate, butyl acetate, ethylene glycol acetate, or amyl acetate), an acid amide (e.g., dimethyl formamide or dimethyl acetanilide), a nitrile (e.g., acetonitrile, propionitrile, or acrylonitrile), a sulfoxide (e.g., dimethyl sulfoxide), and an alcohol ether (e.g., ethylene glycol monomethyl ether or ethylene glycol monoethyl ether).

Examples of the dispersant, the emulsifier, the spreader, and the like include a nonionic surfactant (e.g., a polyoxyethylene alkyl ether, a polyoxyethylene alkyl ester, a polyoxyethylene alkyl phenyl ether, a polyoxyethylene sorbitan alkyl ester, or a sorbitan alkyl ester), an anionic surfactant (e.g., an alkylbenzene sulfonate, an alkyl sulfosuccinate, a polyoxyethylene alkyl sulfate, or an aryl sulfonate), a cationic surfactant (e.g., an alkylamine, a polyoxyethylene alkylamine, or a quaternary ammonium salt), an ampholytic surfactant (e.g., an alkylaminoethylglycine or an alkyldimethylbetaine), polyvinyl alcohol, hydroxypropyl cellulose, carboxymethyl cellulose, gum arabic, tragacanth gum, xanthan gum, polyvinyl acetate, gelatin, casein, and sodium alginate.

The formulation of the plant growth accelerator or the agent for growing vigourous seedlings according to the present invention is not particularly limited. Examples thereof include a wettable powder, a liquid, an oil, a dust, a granule, and a suspension concentrate (flowable).

The amount of a compound represented by formula (1) contained in the plant growth accelerator or the agent for growing vigourous seedlings according to the present invention varies depending on the form of its preparation, the method for its application, and other conditions; however, it is preferably 0.5 to 95% by mass, more preferably 2 to 70% by mass.

The plant growth accelerator or the agent for growing vigourous seedlings according to the present invention can also be used by mixing with any of various well-known conventional agricultural chemicals, such as an agricultural or horticultural fungicide, a plant growth regulator, a insecticide, and a miticide, a fertilizer, or the like.

The plant to which the plant growth accelerator or the agent for growing vigourous seedlings according to the present invention is to be applied is not particularly limited; however, examples thereof include a poaceous cereal crop, such as rice, barley, wheat, Japanese millet, corn, or millet; a cucurbitaceous fruit crop, such as pumpkin, cucumber, melon, water melon, or balsam pear; a cruciferous vegetable, such as turnip, cabbage, Japanese white radish, Chinese cabbage, or rapeseed; a solanaceous vegetable, such as pimento, tomato, eggplant, or red pepper; a vegetable, such as spinach or lettuce; a flower and ornamental plant, such as chrysanthemum, gerbera, pansy, orchid, peony, or tulip; a pulse, such as red bean, kidney bean, soybean, peanut, broad bean, or garden pea; a tuber and root, such as potato, sweet potato, aroid, yam, or taro; and an allium, such as green onion, onion, or shallot.

Among these, a poaceous cereal crop and a cucurbitaceous fruit crop are particularly preferable.

The application method for the plant growth accelerator or the agent for growing vigourous seedlings according to the present invention is not particularly limited. For example, applications are possible, such as application to plants (foliage application), application to the soil in which plants grow (soil application), application to paddy water (submerged application), and application to seeds (seed treatment). Among these, seed treatment is preferable because of having a superior effect in the initial stage of plant growth.

The application amount of the plant growth accelerator or the agent for growing vigourous seedlings according to the present invention can be properly set depending on the plant to which it is to be applied, weather conditions, and the like.

For foliage application, the accelerator or the agent is applied in the form of a solution preferably having an active ingredient concentration of 1 to 10,000 ppm, more preferably 10 to 1,000 ppm, preferably in a volume of 50 to 300 L per 10 are.

For soil and submerged applications, the accelerator or the agent is preferably applied in an amount of 0.1 to 1,000 g, more preferably 10 to 100 g, in terms of an active ingredient per 10 are.

For seed treatment, the accelerator or the agent is preferably applied in an amount of 0.001 to 50 g in terms of an active ingredient per 1 kg of seeds.

The application of the plant growth accelerator or the agent for growing vigourous seedlings according to the present invention enables the vigourous growth of various plants. Particularly, the accelerator or the agent is effective in accelerating the rooting of a plant, growing vigourous seedlings, and increasing the number of tillers and can be expected to increase yield.

### Example

The present invention will now be specifically described with reference to Example. However, the present invention is not intended to be limited to the Example.

### Example 1

Leimay Flowable (trade name, from Nissan Chemical Industries, Ltd.; containing 17.7% amisulbrom) was diluted at the degrees shown in Table 1 or 2 in water to provide chemical solutions according to the present invention.

### Comparative Example 1

Tachigare-Ace Solution (trade name, from Mitsui Chemicals Agro, Inc.; containing 30% hydroxyisoxazole and 4% metalaxyl) was diluted at the degrees shown in Table 1 or 2 in water to provide chemical solutions.

### (Test Example 1) Test on Acceleration of Rice Root Growth and Effect of Growing Vigourous Seedlings

A paper filter was spread in a plastic cup and impregnated with 10 ml of each of the chemical solutions prepared in Example 1 or Comparative Example 1. In non-treatment, water was used in place of the chemical solutions.

In treatment plots, 1 replication was provided for each concentration; in the non-treatment plots, 2 replications were provided. Ten rice seeds (variety: Koshihikari) were sown on each plot of the paper filter and cultivated in a growth chamber at 25°C, and the length of roots was measured after 5 and 7 days. The average of measured values excluding the maximum and minimum values was calculated.

The ratio (%) of the average value in each treatment plot to the average value in the non-treatment plots was calculated. The results are shown in Table 1.

[Table 1]

**Table 1**

| Chemical | | Dilution | Active Ingredient Concentration (ppm) | After 5 Days | | After 7 Days | |
|---|---|---|---|---|---|---|---|
| | | | | Seminal Root Length (cm) | To Non-treatment (%) | Seminal Root Length (cm) | To Non-treatment (%) |
| Amisulbrom Treatment Plot | 1 | 708 | 250 | 2.46 | 120 | 2.91 | 127 |
| | 2 | 2832 | 62.5 | 2.85 | 139 | 4.04 | 176 |
| | 3 | 11328 | 15.6 | 2.65 | 129 | 3.53 | 154 |
| Hydroxyisoxazole/Metalaxyl Treatment Plot | 1 | 1000 | 300/40 | 0.50 | 24 | 0.66 | 29 |
| | 2 | 4000 | 75/10 | 1.15 | 56 | 2.09 | 91 |
| | 3 | 16000 | 18.8/25 | 1.79 | 87 | 2.58 | 112 |
| Non-treatment Plot | 1 | - | - | 2.09 | - | 2.14 | - |
| | 2 | - | - | 2.03 | - | 2.45 | - |
| | Average | | | 2.06 | | 2.29 | |

As shown in Table 1, a significant increasing effect on the length of seminal roots was observed in the rice to which each of the chemical solutions according to the present invention was applied.

### (Test Example 2) Test on Acceleration of Growth of Above-ground/Underground Part of Cucumber and Effect of Growing Vigourous Seedlings

Seedling-raising culture soil for gardening was placed in a 12-cm-by-20-cm plastic pot 10 cm in depth, and 5 seeds of cucumber (Variety:Sagamihanjiro-fushinari) were sown in the soil. Thereinto was uniformly irrigated 500 ml of each chemical solution prepared in Example 1 or Comparative Example 1. Water was used in place of each chemical solution in the non-treatment plots. Each plot has 3 replications. The above-ground and underground parts of the seedlings germinated and grown after 7 days were measured for length. The ratio (%) of the average value in each treatment plot to the average value in the non-treatment plot was calculated. The results are shown in Table 2.

[Table 2]

**Table 2**

| Chemical | Dilution | Active Ingredient Concentration (ppm) | Above-ground Part Length (cm) | To Non-treatment (%) | Underground Part Length (cm) | To Non-treatment (%) |
|---|---|---|---|---|---|---|
| Amisulbrom Treatment Plot | 708 | 250 | 2.2 | 108 | 4.3 | 117 |
| Hydroxyisoxazole/Metalaxyl Treatment Plot | 500 | 600/80 | 1.9 | 91 | 1.5 | 41 |
| Non-treatment Plot | - | - | 2.0 | - | 3.6 | - |

As shown in Table 2, a significant enhancing effect on the length of above-ground and underground parts in the cucumber treated with the chemical solution according to the present invention was observed.

## Claims

1. A plant growth accelerator comprising a compound represented by formula (1) as an active ingredient: 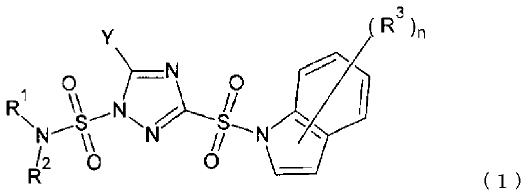 [wherein R¹ and R² each independently represent a C₁₋₄ alkyl group; Y represents a hydrogen atom, a halogeno group, a C₁₋₄ alkyl group, or a C₁₋₄ haloalkyl group; each R³ independently represents a halogeno group, a cyano group, a nitro group, a C₁₋₄ alkyl group, or a C₁₋₄ haloalkyl group; and n represents the number of R³ and is an integer of 0 to 6.]

2. A plant growth accelerator comprising 3-(3-bromo-6-fluoro-2-methylindol-1-ylsulfonyl)-N,N-dimethyl-1H-1,2,4-triazole-1-sulfonamide as an active ingredient.

3. The plant growth accelerator according to claim 1 or 2, wherein the accelerator is for seed treatment.

4. An agent for growing vigourous seedlings, comprising a compound represented by formula (1) as an active ingredient: 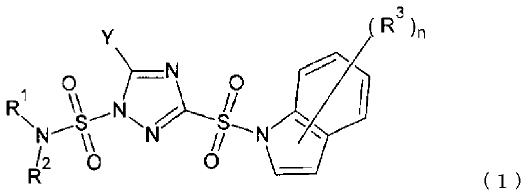 [wherein R¹ and R² each independently represent a C₁₋₄ alkyl group; Y represents a hydrogen atom, a halogeno group, a C₁₋₄ alkyl group, or a C₁₋₄ haloalkyl group; each R³ independently represents a halogeno group, a cyano group, a nitro group, a C₁₋₄ alkyl group, or a C₁₋₄ haloalkyl group; and n represents the number of R³ and is an integer of 0 to 6.]

5. An agent for growing vigourous seedlings, comprising 3-(3-bromo-6-fluoro-2-methylindol-1-ylsulfonyl)-N,N-dimethyl-1H-1,2,4-triazole-1-sulfonamide as an active ingredient.

6. The agent for growing vigourous seedlings according to claim 4 or 5, wherein the agent is for seed treatment.
